# EUROPEAN PATENT SPECIFICATION

(11) **EP 2 153 853 B1**
(45) Date of publication and mention of the grant of the patent: **03.05.2017**
(21) Application number: 08161959.5
(22) Date of filing: 07.08.2008
(51) Int. Cl.: A61L 2/28, G01N 31/22, G01N 21/78, C09D 11/00, C09B 67/00, C09B 29/12, C09B 29/26, C09B 29/00, C09B 29/08

(54) **Composition of indicator for detecting sterilization**
Indikatorzusammensetzung zur Sterilisationsbestimmung
Composition d'indicateur pour la détection de la stérilisation

(43) Date of publication of application: 17.02.2010
(73) Proprietor: Nichiyu Giken Kogyo Co., Ltd., Kawagoe-shi, Saitama 350-1107 (JP)
(72) Inventor: Niwa, Yukiyo, Saitama Saitama 350-1107 (JP)
(74) Representative: dompatent von Kreisler Selting Werner - Partnerschaft von Patent- und Rechtsanwälten mbB

(56) References cited:
- DATABASE WPI Week 200318 Thomson Scientific, London, GB; AN 2003-178531 XP002513360 -& JP 2002 294113 A (SAKURA KUREPASU KK) 9 October 2002 (2002-10-09)
- DATABASE WPI Week 200332 Thomson Scientific, London, GB; AN 2003-336124 XP002513539 -& JP 2002 323451 A (KOBAYASHI N) 8 November 2002 (2002-11-08)
- DATABASE WPI Week 200870 Thomson Scientific, London, GB; AN 2008-L92418 XP002513377 -& JP 2008 132311 A (NICHU GIKEN KOGYO KK) 12 June 2008 (2008-06-12)
- DATABASE WPI Week 200333 Thomson Scientific, London, GB; AN 2003-346504 XP002513359 -& JP 2003 004638 A (KOBAYASHI N) 8 January 2003 (2003-01-08)

## Description

### Technical Field

The present invention relates to a composition used for a sterilization indicator that detects completion of high-pressure steam sterilization of pharmaceuticals and ethylene oxide gas sterilization of medical devices.

### Background Art

Because the pharmaceuticals, the medical devices and the like for an intervention are used under an aseptic condition, they are sterilized preliminary. The pharmaceuticals such as infusions and sanitary materials such as gauze are sterilized in an autoclave under high-pressure steam atmosphere, while the medical devices such as a catheter, an injection needle, a probe of an examination equipment are sterilized under ethylene oxide gas atmosphere.

In order to determine whether the sterilization is fully completed, a chemical indicator discolored upon a reaction with the high-pressure steam or the ethylene oxide gas is arranged with products to be sterilized such as the pharmaceuticals and the medical devices, and the color change of the chemical indicator is visually observed for the determination.

As a composition for a sterilization indicator discolored upon the reaction with both of the high-pressure steam and the ethylene oxide gas, Japanese Patent Provisional Publication No.03-138864 discloses a composition including a monoazo dye, and an organic acid or a metal salt thereof. When rapid high-pressure steam sterilization is detected using this composition, only the sterilization under a highly-limited condition can be detected. Because the polar organic acid and the metal salt thereof, which are necessary to be included in the composition in large amounts for sublimating the monoazo dye to discolor thereof, are hard to disperse in nonpolar binders and solvents thereof and thus hard to be applied onto a substrate, the composition to be ink cannot be prepared easily.

As a composition for a sterilization indicator discolored upon a reaction with both of the high-pressure steam and the ethylene oxide gas, Japanese Patent Provisional Publication No.3-138865 discloses a composition including a transition metal compound, a sulfur compound or sulfur, a monoazo dye, and an organic acid or a metal salt thereof, while Japanese Patent Provisional Publication No.2002-322315 discloses a composition including nicotinamide, isonicotinic acid hydrazide, a bismuth compound, sulfur or a sulfur compound, a polyamide-nitrocellulose resin and an acrylic-cellulose resin.

JP 2002-294113 **discloses an ink for detecting ethylene oxide gas sterilization. Said ink essentially contains an azo dye which is selected from among specific azo dyes. A sterilization indicator pigment composition comprising disperse dyes, an organic acid, a bismuth compound, sulfur or sulfur compound, urethane/nitrocellulose resin, polyamide/nitrocellulose resin, and acryl/alkyd/nitrocellulose resin is disclosed in** JP 2003-323451**.**

As a composition for a sterilization indicator discolored upon a reaction not only with high-pressure steam and ethylene oxide gas but also hydrogen peroxide low-temperature plasma and formalin, Japanese Patent Provisional Publication No.2002-323451 discloses a composition including a bismuth compound, a sulfur compound including sulfur, an organic acid compound, an urethane-nitrocellutose resin, a polyamide-nitrocellulose resin and an acrylic-alkyd-nitrocellulose resin. Especially during high-pressure steam sterilization, these compositions disclosed in Japanese Patent Provisional Publication Nos. 3-138865, 2002-322315 and 2002-323451 cause sulfurous smell derived from the sulfur or the sulfur compound included therein. Also, the color hues of these compositions change from chromatic color to between dark color and black color that is relatively difficult to be visually observed.

### Disclosure of Invention

The present invention has been developed to solve the foregoing problems. It is an object of the present invention to provide a simple and sanitary composition of a sterilization indicator for detecting completion of sterilization by clearly changing the color hue thereof without any bad smell, which is used mainly for high-pressure steam sterilization and where necessary simply applicable for ethylene oxide gas sterilization. Also, it is another object of the present invention to provide a sterilization indicator to which the composition is firmly applied.

The composition of the sterilization indicator for detecting sterilization of the present invention developed for accomplishing the foregoing objects comprises a saccharide and a monoazo dye, **as disclosed in claim 1. Preferred embodiments are mentioned in the dependent claims.**

The monoazo dye of the composition of the sterilization indicator is benzathiazoleazobenzenes and/or azobenzenes.

The monoazo dye is at least one selected from the group consisting of the benzothiazoleazobenzenes represented by the following chemical formula (I)

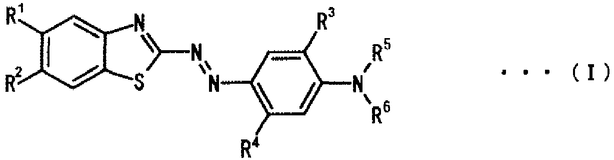

(in the chemical formula (I), R¹ is selected from the group consisting of a hydrogen atom, a hydroxyl group, an alkylsulfo group having 1 to 4 carbon atoms, an alkoxyl group having 1 to 4 carbon atoms, a halogen group, an aminoacyl group and nitro group; R² is selected from the group consisting of a hydrogen atom, a hydroxyl group, an alkylsulfo group having 1 to 4 carbon atoms, a nitrate, an alkyl group having 1 to 4 carbon atoms, an acyloxy-substituted alkyl group having a principle-chain thereof of 1 to 4 carbon atoms, a cyano-substituted alkyl group having a principle-chain thereof of 1 to 4 carbon atoms, an alkoxy-substituted alkyl group having a principle-chain thereof of 1 to 4 carbon atoms and a halogen group; R³ and R⁴ are the same or different to each other and selected from the group consisting of a hydrogen atom, a hydroxyl group, an alkyl group having 1 to 4 carbon atoms, an alkoxyl group having 1 to 4 carbon atoms and an acylamino group; R⁵ and R⁶ are the same or different to each other and selected from the group consisting of a hydrogen atom, a hydroxyl group, a nitrate, an alkyl group having 1 to 4 carbon atoms, an acyloxy-substituted alkyl group having a principle-chain thereof of 1 to 4 carbon atoms, a cyano-substituted alkyl group having a principle-chain thereof of 1 to 4 carbon atoms, an alkoxy-substituted alkyl group having a principle-chain thereof of 1 to 4 carbon atoms and a hydroxy-substituted alkyl group having a principle-chain thereof of 1 to 4 carbon atoms),
and the azobenzenes represented by the following chemical formula (II) 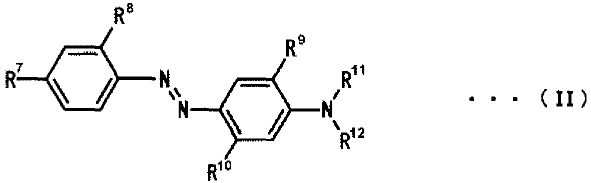 (in the chemical formula (II), R⁷ and R⁸ are the same or different to each other and selected from the group consisting of a hydrogen atom, a hydroxyl group, a nitro group, a cyano group, a halogen group and an acylamino group; R⁹ and R¹⁰ are the same or different to each other and selected from the group consisting of a hydrogen atom, a hydroxyl group and an alkyl group having 1 to 4 carbon atoms; R¹¹ and R¹² are the same or different to each other and selected from the group consisting of a hydrogen atom, a hydroxyl group, an alkyl group having 1 to 4 carbon atoms, a phenyl group, a cyano-substituted alkyl group having a principle-chain thereof of 1 to 4 carbon atoms, a hydroxyl-substituted alkyl group having a principle-chain thereof of 1 to 4 carbon atoms and an acyloxy-substituted alkyl group having a principle-chain thereof of 1 to 4 carbon atoms.)

The saccharide is a reducing monosaccharide having at least a free aldehyde group or a hemiacetal group, or a polysaccharide having the monosaccharide as a repeating unit.

The saccharide is selected from the group consisting of starch, dextrin, alginic acid, carrageenan, gum arabic, agar, agarose, galactan, pectin, glucose, galactose, mannose, arabinose and a derivative thereof.

The derivative is salt or ester.

The composition of the sterilization indicator further comprises an organic acid.

The organic acid is an aliphatic carboxylic acid or an aromatic carboxylic acid.

The sterilization indicator comprises a color-changing layer of the applied composition of the sterilization indicator onto a substrate or a product to be sterilized.

The substrate is made from paper or plastic and in a shape of a card, a tape, a sheet or a bag.

The color-changing layer is covered with a protective layer made of plastic film.

The composition of the sterilization indicator of the present invention sensitively and clearly discolors to a different color hue, which can be easily and visually observed, upon a reaction with either high-pressure steam or ethylene oxide gas. Depending on a sterilization condition of sterilization time, temperature of the high-pressure steam or concentration of the ethylene oxide gas, the composition discolors to a certain different color hue. Also, because the composition does not include sulfur and a sulfur compound, it does not cause sulfurous smell derived therefrom. Therefore, the composition is sanitary.

The sterilization indicator comprising such composition can be used for detecting completion of the sterilization by either of the high-pressure steam or the ethylene oxide gas. Therefore, it is convenient as it is unnecessary to prepare a different indicator for each sterilization method. In addition, by printing with or applying the composition to a substrate, the indicator comprising a firmly formed sterilization color-changing layer can be prepared easily and efficiently. Therefore, it can be prepared with high yield and thus less expensive and economical.

When the completion of the sterilization is detected by using the indicator, the color-changing layer thereof discolors to a clear and visible color hue having significant color difference with the previous color thereof, depending on sterilization time, temperature of the high-pressure steam sterilization or concentration of the ethylene oxide gas.

### Brief Description of Drawings

Fig. 1 is a type cross-sectional view of the sterilization indicator of the present invention.
Fig. 2 is a type cross-sectional view of the sterilization indicator of the present invention.

### Explanations of Numeral References

1 is a sterilization indicator, 4 is a color-changing layer, 5 is a substrate of the indicator, 7 is an adhesive layer, 9 is a plastic protective layer **9**.

Hereunder, embodiments of the present invention are explained in detail. However, it is not intended to be limited to these embodiments.

Embodiment of preparing the composition of the sterilization indicator to which the present invention is applied is explained.

The composition of the sterilization indicator is prepared by homogeneously kneading a monoazo dye as colorant and a saccharide, and if necessary a solvent, a resin or an ink vehicle including thereof, using a kneader.

To 100 parts by weight of the ink vehicle including an ink components such as the solvent and the resin, 0,01 to 50 parts by weight, preferably 0.01 to 5 parts by weight, of the monoazo dye and 0.1 to 50 parts by weight of the saccharide can be used.

The saccharide **is** a reducing monosaccharide having **at least** a free aldehyde group or a hemiacetal group, **or** a polysaccharide having the reducing monosaccharide as a repeating unit, or **it can be** the polysaccharide having the reducing monosaccharide and another monosaccharide as further repeating units.

Example of the monosaccharide is aldohexose such as glucose, galactose, mannose, arabinose and so on, or a derivative thereof.

It is preferable that the polysaccharide is soluble a polysaccharide into cold water or hot water and is especially natural polysaccharide. Such water soluble polysaccharide is highly safe. Also, during high-pressure steam sterilization, such polysaccharide acts on keeping the high-pressure steam in the color-changing layer, and within the temperature range thereof, hydrolysis reaction tends to be performed partially.

Concrete examples of the polysaccharide are a polysaccharide having a group bonding of a reducing group of a monosaccharide and an alcoholic hydroxyl group of another monosaccharide. Concrete examples thereof are starch, dextrin, alginic acid, carrageenan, gum arabic, agar or agarose as one component thereof, galactan and pectin. The polysaccharide can be a derivative thereof.

It is preferable that the polysaccharide is carrageenan, agar or alginic acid that has a sulfonic group or a carboxyl group as an acidic group within the molecular structure thereof. Even if the organic acid is included in the composition only in small amounts, the acidic group within the molecular structure accomplishes to perform the hydrolysis reaction with the polysaccharide by the high-pressure steam easily. Therefore, the reaction is performed well and thus the composition discolors upon the reaction sensitively.

The saccharide can be salt or ester. Concrete examples thereof are sodium alginate, potassium alginate, calcium alginate and alginic acid ester.

The monoazo dye is the colorant that discolors upon the reaction with the monosaccharide comprising the free aldehyde group, the monosaccharide comprising the hemiacetal group or the partially hydrolyzed polysaccharide under the high-pressure steam atmosphere, or is other colorant that discolors upon the reaction with the ethylene oxide gas.

It is preferable that the monoazo dye is a compound bonded with an aromatic ring and an aromatic ring/a heterocyclic ring by a monoazo group, preferably a compound bonded with a benzene ring that may have a substituent and another benzene ring that may have a substituent or a benzothiazole ring, through a monoazo group. It is furthermore preferable that the monoazo dye is the one represented by the chemical formula (I) and (II), Concrete examples thereof are C.I. (Color Index) Disperse Red 58, C.I. Disperse Red 88, C.I. Disperse Red 110, C.I. Disperse Red 117, C.I. Disperse Red 137, C.I. Disperse Violet 38, C.I. Disperse Violet 43, C.I. Disperse Blue 102, Disperse Red 152, Disperse Red 153 and Disperse Red 154.

If the amount of the monoazo dye in the composition is less than 0.01 parts by weight, the color hue of the color-changing layer formed by the composition is diluted. On the other hand, if the amount thereof is more than 5 parts by weight, the color hue thereof discolors too thick, and as a result, it is difficult to visually observe a slight change of the color hue.

As an accelerator agent for a reaction of the monoazo dye and the saccharide and also for a hydrolysis reaction of the polysaccharide, the sterilization indicator may include the organic acid at most of 50 parts by weight. Example of the organic acid is a carboxylic acid that is not a weak acid and has lower acidity compared to that of hydrochloric acid, sulfuric acid and nitric acid.

Examples of the carboxylic acid are an aliphatic carboxylic acid, an aromatic carboxylic acid, and a derivative thereof. Examples thereof are a saturated or unsaturated aliphatic carboxylic acid having a substituent such as an aryl group, an acyl group, a hydroxy group, an alkoxy group, an amino group and an oxo group, or the salt thereof, or an aromatic carboxylic acid having a substituent such as an alkyl group, an acyl group, a hydroxy group, an alkoxy group, an amino group or an oxo group, or the salt thereof.

Examples of the aliphatic carboxylic acid or the derivative thereof are acrylic acid, adipic acid, L-asparagine acid, azelaic acid, itaconic acid, undecylenic acid, citric acid, glutaconic acid, diglycol acid, DL-tartaric acid, sebacic acid, sorbic acid, naphthene acid, hippuric acid, barbituric acid, pivalic acid, pyruvic acid, fumaric acid, benzilic acid, maleic acid, malonic acid and methacrylic acid.

Examples of the aromatic carboxylic acid or the derivative thereof are an aromatic monocarboxylic acid such as benzoic acid, methylbenzoic acid, dimethylbenzoic acid, 2,3-dimethylbenzoic acid, 3,5-dimethylbenzoic acid, 2,3,4-trimethylbenzoic acid, 2,3,5-trimethylbenzoic acid, 2,4,5-trimethylbenzoic acid, 2,4,6-trimethylbenzoic acid, 3,4,5-trimethylbenzoic acid, 2-hydroxybenzoic acid, methoxybenzoic acid, hydroxy(methyl)benzoic acid, 2-hydroxy-3-methylbenzoic acid, 2-hydroxy-4-methylbenzoic acid, 2-hydroxy-5-methylbenzoic acid, 2,3-dihydroxybenzlic acid, 2,4-dihydroxybenzlic acid, 2,5-dihydroxybenzoic acid, 2,6-dihydroxybenzoic acid, 3,4-dihydroxybenzoic acid, 3,5-dihydroxybenzoic acid, 4-hydroxy-3-methoxybenzoic acid, 3-hydroxy-4-methoxybenzoic acid, 3,4-dimethoxybenzoic acid, 2,3-dimethoxybenzoic acid, 2,4-dihydroxy-6-methylbenzoic acid, 3,4,5-trihydroxybenzoic acid, 4-hydroxy-3,5-dimethylbenzoic acid, 2,4,5-trimethoxybenzoic acid, 2-(carboxymethyl)benzoic acid, 3-(carboxymethyl)benzoic acid, 4-(carboxymethyl)benzoic acid, 2-(carboxycarbonyl)benzoic acid, 3-(carboxycarbonyl)benzoic acid, 4-(carboxycarbonyl)benzoic acid, anthranilic acid, salicylic acid, nicotinic acid; an aromatic polycarboxylic acid such as phthalic acid, isophthalic acid, terephthalic acid, hemimellitic acid, trimellitic acid and trimesic acid.

The ink vehicle includes a resin that does not lose a color-changing property of the indicator and has a sufficient adhesive property to a substrate. Examples thereof are ethyl cellulose, nitrocellulose, a butyral resin, an acrylic resin such as an acrylic ester resin and a methacrylic acid ester resin, a polyamide resin, a phenol resin and a modified rosin maleic acid resin. These may include solvent and be a commercially-available ink vehicle.

The composition of the sterilization indicator may further comprise an additive used for a general indicator composition such as a colored pigment, a rust inhibitor, a surface-active agent and an auxochrome agent.

When the composition of the sterilization indicator includes silica, barium sulfate, clay, magnesium carbonate, calcium carbonate and so on as the additive, permeability of the high-pressure steam or the ethylene oxide gas is improved. When the composition comprises the pigment with high heat resistance or gas resistance, the color hue thereof around the sterilization can be easily adjusted for better visual observation.

If necessary, a solvent is used together with the resin in order for the composition of the sterilization indicator to accomplish better printing performance. The solvent may include an organic solvent such as mineral spirit, solvent naphtha, methyl alcohol, isopropyl alcohol, methyl ethyl ketone, methyl isobutyl ketone, cyclohexanone, ethyl acetate, propyl acetate, isobutyl acetate, isoamyl acetate, butyl cellosolve, toluene, xylene and so on; tung oil, linseed oil, soybean oil, castor oil, a dry oil and the mixture thereof. These can be used solely or plurally.

The composition of the sterilization indicator can be prepared as an ink by being kneaded using a kneader such as a ball mill, a roll mill, a sand mill and so on.

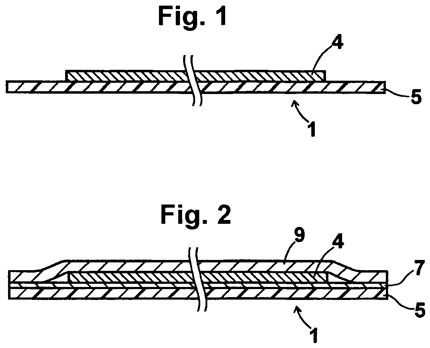

Hereunder, examples of manufacturing a sterilization indicator using the composition are explained referring to Fig. 1.

The composition of the sterilization indicator is printed on an indicator substrate 5 to form a color-changing layer 4, and then a sterilization indicator 1 is obtained.

The sterilization indicator is printed on the substrate made from paper such as high-quality paper or crepe paper, the substrate made from plastic, synthetic paper, fabric or nonwoven textile such as a card, a tape, a sheet and a bag;
or the substrate for packaging the product to be sterilized made therefrom such as a paper-made packaging sheet and paper-made packaging bag. The sterilization indicator may be directly printed to the product to be sterilized.

The sterilization indicator is used as follows.

Products to be sterilized are put in an autoclave to set therein, and the sterilization indicator in a shape of a card is placed therein. The autoclave is rocked and filled with high-pressure steam in order to sterilize the products. As a result, the monoazo dye of the color-changing layer is reacted with the high-pressure steam and then discolors.

When the products are sterilized by the ethylene oxide gas in a tank for sterilization instead of by the high-pressure steam sterilization, the monoazo dye in a color-changing layer of the sterilization indicator is reacted with the ethylene oxide gas and then discolors.

Although it is still not fully explained about a mechanism that the color-changing layer, to which the composition of the sterilization indicator was printed, changes the color hue under the high-pressure steam atmosphere or the ethylene oxide gas atmosphere, it is assumed as follows.

A sterilization indicator comprising a color-changing layer prepared by the composition that includes the monoazo dye represented by the chemical formula (I) and the organic acid is explained as an example. When the indicator is exposed to high-pressure steam, proton derived from the organic acid in the presence of steam reacts to the monosaccharide. As a result, nitrogen of a monoazo group of the monoazo dye and an aldehyde group of the monosaccharide react through the proton as a catalyst. It results in changing the density of electrons of the monoazo dye, and therefore the color-changing layer discolors for example from red to green.

A sterilization indicator comprising a color-changing layer prepared by the composition that includes starch as the polysaccharide having principle components of amylase of alpha-1,4 glycosidical-bonded D-glucose and amylopection of alpha-1,6 glycosidical-bonded D-glucose, the monoazo dye represented by the chemical formula (I), and the organic acid is explained as another example. When the indicator is exposed to high-pressure steam, proton derived from the organic acid in the presence of steam reacts to the polysaccharide. As a result, a glucopyranose group as a glucose cyclic isomer, which is partially polysaccharide, opens circular by being hydrolyzed and then produces an aldehyde group. Nitrogen of a monoazo group of the monoazo dye and the produced aldehyde group react through the proton as a catalyst. It results in changing the density of electrons of the monoazo dye, and therefore the color-changing layer discolors for example from red to green.

On the other hand, when the indicator is exposed to ethylene oxide gas, the ethylene oxide gas reacts to the monoazo dye that results the change of resonance structure thereof. Moreover, as the change of color is accelerated by proton generated due to the coexistence of the organic acid, the color-changing layer discolors for example from red to blue.

Incidentally, depending on a sterilization condition of the product to be sterilized, the discolored color hue, thickness of the color and discoloring speed can be adjusted by controlling sorts or concentration of the saccharide, the monoazo dye and the organic acid in the composition.

A sterilization indicator 1 to which the present invention is applied is shown in Fig. 2. As shown in Fig. 2, a color-changing layer 4 of the sterilization indicator 1 may be covered by a plastic protective layer 9 through an adhesive layer 7 because this enables to prevent the composition of the indicator and the compound of the composition from being contaminated due to a contact with a product to be sterilized.

Hereunder, examples of preparing the composition of the sterilization indicator of the present invention and the sterilization indicator using thereof are explained. Also, comparative examples of preparing the composition to which the present invention is not applied and the sterilization indicator using thereof are explained.

### (Examples 1A to 1I and Comparative Example 1a)

By kneading the compounds for Examples 1A to 1I and Comparative Example 1a shown in Table 1 in a ball mill for 24 hours, each ink was obtained as a composition for a sterilization indicator used for a screen printing. The sterilization indicators printed with the obtained ink on films were sterilized by high-pressure steam at 134C° for 8 minutes. The color hue of the indicators prepared by the ink of Examples 1A to 1I discolored from red to green.

The sterilization indicators printed with the obtained ink on films were sterilized in a constant temperature oven at 137C° for 30 minutes. The color hue of the indicators of Examples 1A to 1I and Comparative Example 1a stayed as red.

Other sterilization indicators similarly printed onto high-quality paper with the obtained ink were sterilized by ethylene oxide gas for 240 minutes. The sterilization condition thereof was the ethylene oxide gas with 20% of carbon dioxide gas; 100kPa of feed gas; 50C° of temperature; 50% of relative humidity. The color hue of the indicators of Examples 1A to 1I and Comparative Example 1a discolored from red to blue after a lapse of 120 to 240 minutes.

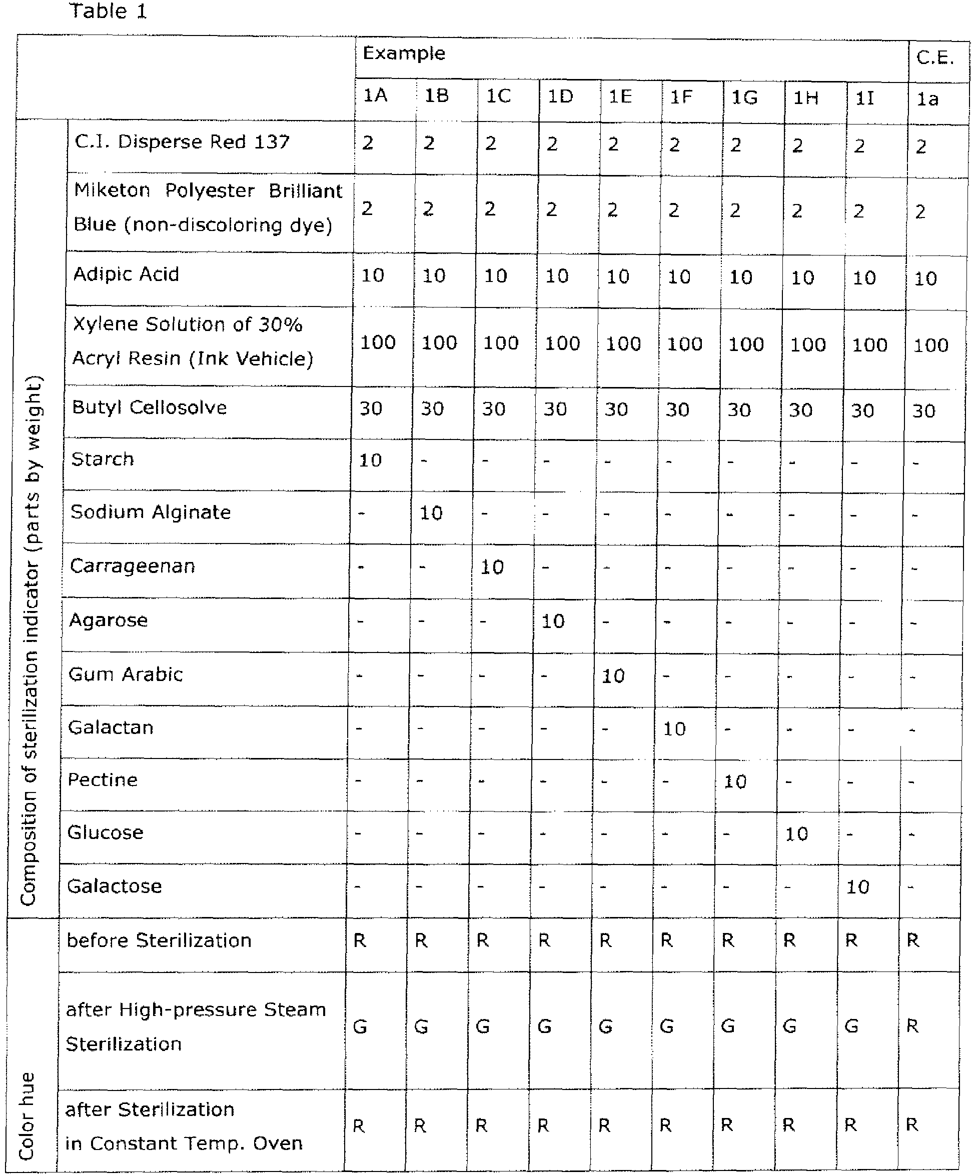

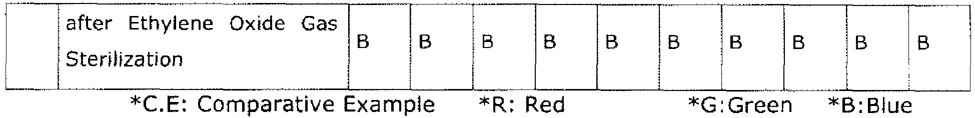

### (Examples 2A to 2B and Comparative Example 2a)

By kneading the compounds for Examples 2A, 2B and Comparative Example 2a shown in Table 2 in a ball mill for 24 hours, each ink was obtained as a composition for a sterilization indicator used for a screen printing. The sterilization indicators printed with the obtained ink on films were sterilized by high-pressure steam at 134C° for 1 minute. The color hue of the indicators prepared by the ink of Examples 2A and 2B discolored from red to green.

The sterilization indicators printed with the obtained ink on films were sterilized in a constant temperature oven at 137C° for 30 minutes. The color hue of the indicators of Examples 2A, 2B and Comparative Example 2a stayed as red.

Other sterilization indicators similarly printed onto high-quality paper with the prepared ink were sterilized by ethylene oxide gas for 240 minutes. The sterilization condition thereof was ethylene oxide gas with 20% of carbon dioxide gas; 100kPa of feed gas; 50C° of temperature; 50% of relative humidity. The color hue of the indicators of Examples 2A, 2B and Comparative Example 2a discolored from red to blue after a lapse of 120 to 240 minutes.

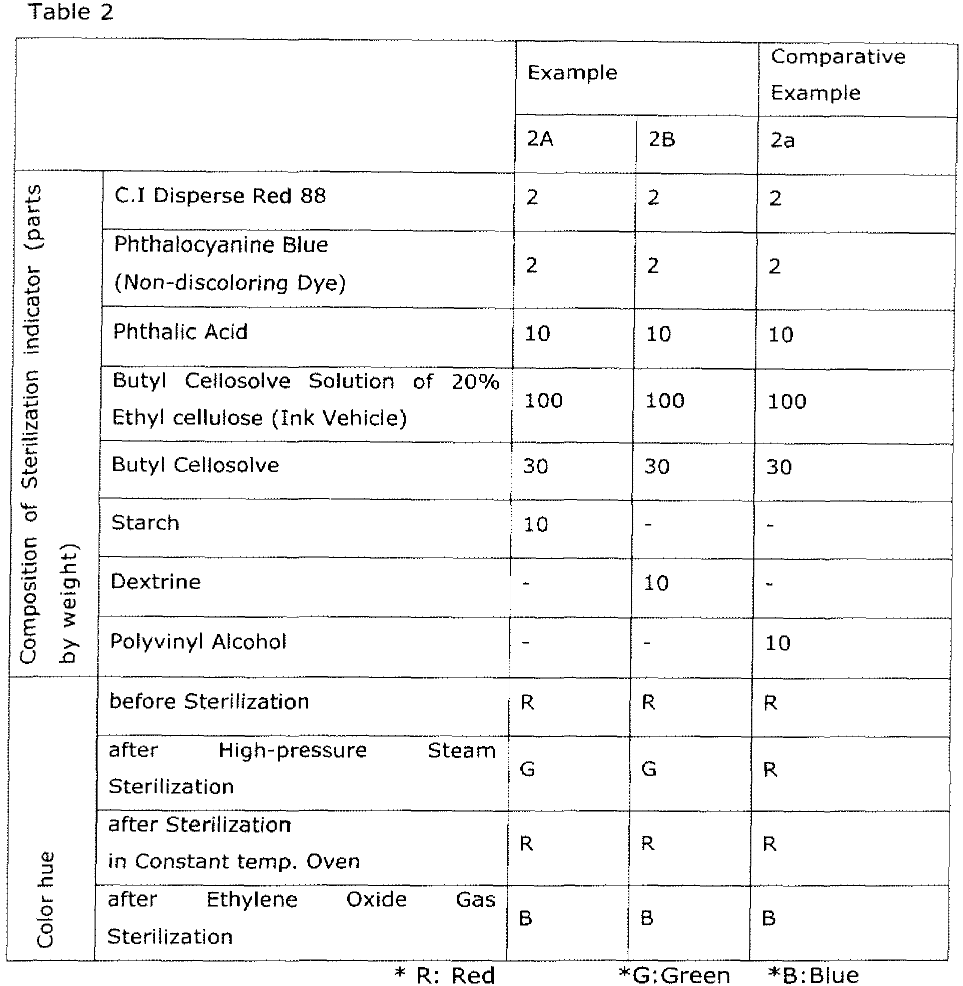

### (Example 3A and Comparative Example 3a)

By kneading the compounds for Example 3A and Comparative Example 3a shown in Table 3 in a ball mill for 24 hours, each ink was obtained as a composition for a sterilization indicator used for a screen printing. The sterilization indicators printed with the obtained ink on films were sterilized by high-pressure steam at 134C° for 1 minute. The color hue of the indicator prepared by the ink of Example 3A discolored from red to green.

The sterilization indicators printed with the obtained ink on films were sterilized in a constant temperature oven at 137C° for 30 minutes. The color hue of both the indicators of Example 3A and Comparative Example 3a stayed as red.

Other sterilization indicators similarly printed onto high-quality paper with the obtained ink were sterilized by ethylene oxide gas for 240 minutes. The sterilization condition thereof was ethylene oxide gas with 20% of carbon dioxide gas; 100kPa of feed gas; 50C° of temperature; 50% of relative humidity. The color hue of the indicator of Example 3A discolored from red to blue after a lapse of 120 to 240 minutes.

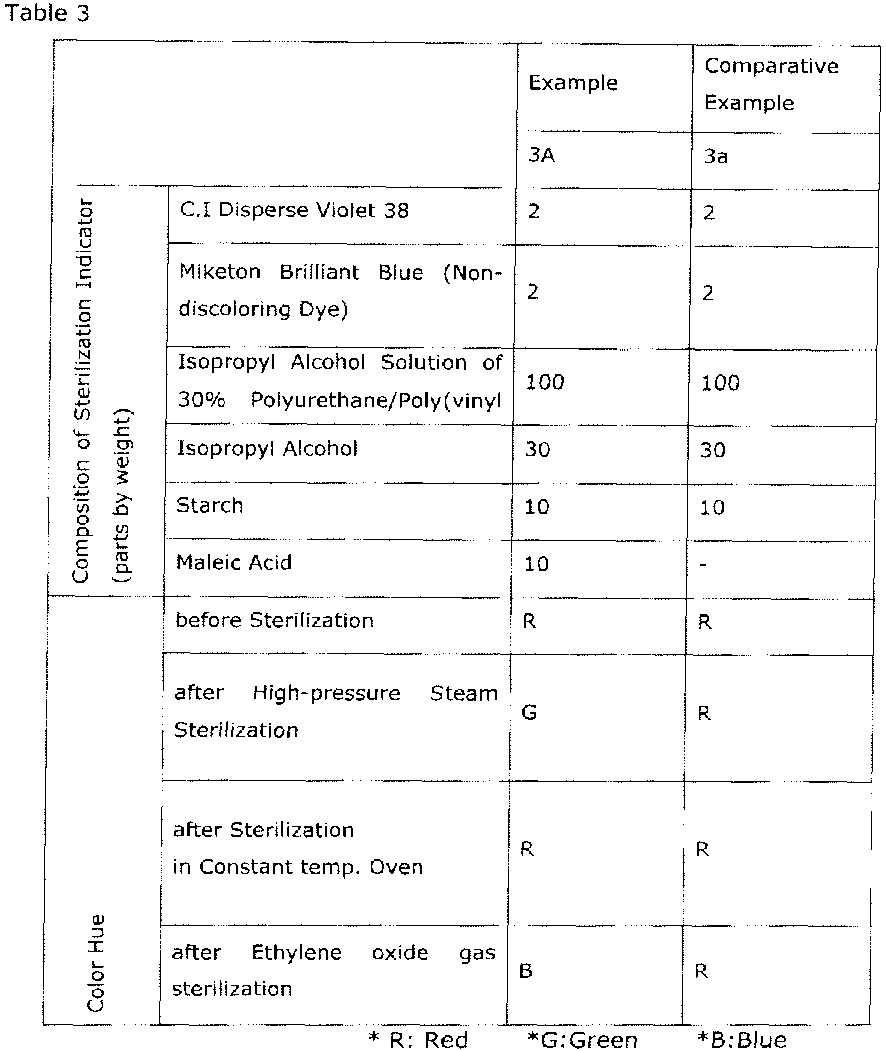

As shown in Table 1 to 3, the color hue of the sterilization indicator to which the present invention is applied, clearly discolors within the prescribed sterilization time by responding to the high-pressure steam and the ethylene oxide gas.

### Industrial Applicability

The composition of the sterilization indicator of the present invention is useful for manufacturing the indicator for detecting the completion of sterilization of pharmaceuticals, medical devices and processed foods such as pouch-packed foods.

Also, the sterilization indicator comprising the color-changing layer prepared by printing with the ink of this composition is useful for detecting the completing of the sterilization by the high-pressure steam and the ethylene oxide gas.

The sterilization indicator is prepared in shape of a card with a print of a number, a letter or a design pattern as a color changing layer, and it is used for detecting completion of the sterilization or being kept as a proof thereof.

## Claims

1. A composition of a sterilization indicator for usage of detecting sterilization by either high-pressure steam or ethylene oxide gas comprising compounds for discoloring to a different color hue, said composition comprises:
a reducing saccharide selected from the group consisting of a reducing monosaccharide having at least a free aldehyde group or a hemiacetal group and a water-soluble polysaccharide having a reducing monosaccharide as a repeating unit,
a monoazo dye at least one selected from the group consisting of benzothiazoleazobenzenes and azobenzenes,
an organic acid and an ink vehicle including at least one selected from the group consisting of ethyl cellulose, nitrocellulose, a butyral resin, an acrylic resin, a polyamide resin, a phenol resin and a modified rosin maleic acid resin.

2. The composition of the sterilization indicator according to claim 1, wherein the monoazo dye is at least one selected from the group consisting of the benzothiazoleazobenzenes represented by the following chemical formula (I) 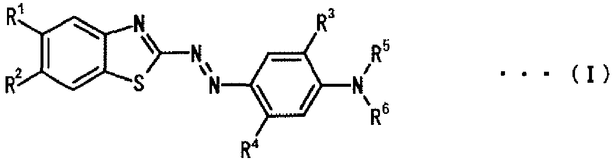 in the chemical formula (I), R¹ is selected from the group consisting of a hydrogen atom, a hydroxyl group, an alkylsulfo group having 1 to 4 carbon atoms, an alkoxyl group having 1 to 4 carbon atoms, a halogen group, an aminoacyl group and nitro group; R² is selected from the group consisting of a hydrogen atom, a hydroxyl group, an alkylsulfo group having 1 to 4 carbon atoms, a nitrate, an alkyl group having 1 to 4 carbon atoms, an acyloxy-substituted alkyl group having a principle-chain thereof of 1 to 4 carbon atoms, a cyano-substituted alkyl group having a principle-chain thereof of 1 to 4 carbon atoms, an alkoxy-substituted alkyl group having a principle-chain thereof of 1 to 4 carbon atoms and a halogen group; R³ and R⁴ are the same or different to each other and selected from the group consisting of a hydrogen atom, a hydroxyl group, an alkyl group having 1 to 4 carbon atoms, an alkoxyl group having 1 to 4 carbon atoms and an acylamino group; R⁵ and R⁶ are the same or different to each other and selected from the group consisting of a hydrogen atom, a hydroxyl group, a nitrate, an alkyl group having 1 to 4 carbon atoms, an acyloxy-substituted alkyl group having a principle-chain thereof of 1 to 4 carbon atoms, a cyano-substituted alkyl group having a principle-chain thereof of 1 to 4 carbon atoms, an alkoxy-substituted alkyl group having a principle-chain thereof of 1 to 4 carbon atoms and a hydroxy-substituted alkyl group having a principle-chain thereof of 1 to 4 carbon atoms;
and the azobenzenes represented by the following chemical formula (II) 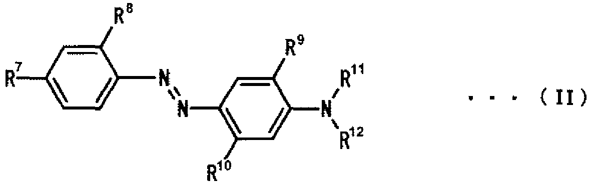 in the chemical formula (II), R⁷ and R⁸ are the same or different to each other and selected from the group consisting of a hydrogen atom, a hydroxyl group, a nitro group, a cyano group, a halogen group and an acylamino group; R⁹ and R¹⁰ are the same or different to each other and selected from the group consisting of a hydrogen atom, a hydroxyl group and an alkyl group having 1 to 4 carbon atoms; R¹¹ and R¹² are the same or different to each other and selected from the group consisting of a hydrogen atom, a hydroxyl group, an alkyl group having 1 to 4 carbon atoms, a phenyl group, a cyano-substituted alkyl group having a principle-chain thereof of 1 to 4 carbon atoms, a hydroxyl-substituted alkyl group having a principle-chain thereof of 1 to 4 carbon atoms and an acyloxy-substituted alkyl group having a principle-chain thereof of 1 to 4 carbon atoms.

3. The composition of the sterilization indicator according to claim 1, wherein the reducing saccharide is selected from the group consisting of starch, dextrin, alginic acid, carrageenan, gum arabic, agar, agarose, galactan, pectin, glucose, galactose, mannose and arabinose.

4. The composition of the sterilization indicator according to claim 3, wherein the derivative is salt or ester.

5. The composition of the sterilization indicator according to claim 1, wherein the polysaccharide is carrageenan, agar or alginic acid that has a sulfonic group or a carboxyl group within the molecular structure thereof.

6. The composition of the sterilization indicator according to claim 1, wherein the organic acid is an aliphatic carboxylic acid or an aromatic carboxylic acid.

7. A sterilization indicator comprising a color-changing layer of the applied composition of the sterilization indicator according to claim 1 onto a substrate or a product to be sterilized.

8. The sterilization indicator according to claim 7, wherein the substrate is made from paper or plastic and in a shape of a card, a tape, a sheet or a bag.

9. The sterilization indicator according to claim 7, wherein the color-changing layer is covered with a protective layer made of plastic film.

10. Use of the composition of the sterilization indicator according to claim 1 for detecting the completing of the sterilization by the high-pressure steam and the ethylene oxide gas.

## Patentansprüche

1. Zusammensetzung mit einem Sterilisationsindikator zur Verwendung beim Nachweis der Sterilisation entweder durch Hochdruckdampf oder durch Ethylenoxidgas, umfassend Verbindungen zur Verfärbung nach einem anderen Farbton, wobei die Zusammensetzung umfasst:
ein reduzierendes Saccharid, das aus der Gruppe ausgewählt ist, die aus einem reduzierenden Monosaccharid, das wenigstens eine freie Aldehydgruppe oder eine Hemiacetalgruppe aufweist, und einem wasserlöslichen Polysaccharid, das ein reduzierendes Monosaccharid als Repetiereinheit aufweist, besteht;
einen Monoazofarbstoff, der wenigstens einen umfasst, der aus der Gruppe ausgewählt ist, die aus Benzothiazolazobenzolen und Azobenzolen besteht;
eine organische Säure; und
einen Tintenträger, der wenigstens einen umfasst, der aus der Gruppe ausgewählt ist, die aus Ethylcellulose, Nitrocellulose, einem Butyralharz, einem Acrylharz, einem Polyamidharz, einem Phenolharz und einem modifizierten Kolophonium-Maleinsäure-Harz besteht.

2. Zusammensetzung mit dem Sterilisationsindikator gemäß Anspruch 1, wobei der Monoazofarbstoff wenigstens einer ist, der aus der Gruppe ausgewählt ist, die aus folgenden besteht:
den Benzothiazolazobenzolen, die durch die folgende chemische Formel (I) dargestellt werden: 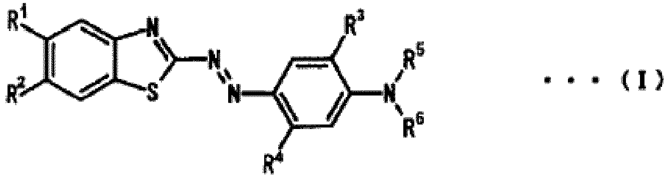 wobei in der chemischen Formel (I) R¹ aus der Gruppe ausgewählt ist, die aus einem Wasserstoffatom, einer Hydroxygruppe, einer Alkylsulfogruppe mit 1 bis 4 Kohlenstoffatomen, einer Alkoxygruppe mit 1 bis 4 Kohlenstoffatomen, einer Halogengruppe, einer Aminoacylgruppe und einer Nitrogruppe besteht; R² aus der Gruppe ausgewählt ist, die aus einem Wasserstoffatom, einer Hydroxygruppe, einer Alkylsulfogruppe mit 1 bis 4 Kohlenstoffatomen, einem Nitrat, einer Alkylgruppe mit 1 bis 4 Kohlenstoffatomen, einer acyloxysubstituierten Alkylgruppe, deren Hauptkette 1 bis 4 Kohlenstoffatome aufweist, einer cyanosubstituierten Alkylgruppe, deren Hauptkette 1 bis 4 Kohlenstoffatome aufweist, einer alkoxysubstituierten Alkylgruppe, deren Hauptkette 1 bis 4 Kohlenstoffatome aufweist, und einer Halogengruppe besteht; R³ und R⁴ gleich oder verschieden sind und aus der Gruppe ausgewählt sind, die aus einem Wasserstoffatom, einer Hydroxygruppe, einer Alkylgruppe mit 1 bis 4 Kohlenstoffatomen, einer Alkoxygruppe mit 1 bis 4 Kohlenstoffatomen, und einer Acylaminogruppe besteht; R⁵ und R⁶ gleich oder verschieden sind und aus der Gruppe ausgewählt sind, die aus einem Wasserstoffatom, einer Hydroxygruppe, einem Nitrat, einer Alkylgruppe mit 1 bis 4 Kohlenstoffatomen, einer acyloxysubstituierten Alkylgruppe, deren Hauptkette 1 bis 4 Kohlenstoffatome aufweist, einer cyanosubstituierten Alkylgruppe, deren Hauptkette 1 bis 4 Kohlenstoffatome aufweist, einer alkoxysubstituierten Alkylgruppe, deren Hauptkette 1 bis 4 Kohlenstoffatome aufweist, und einer hydroxysubstituierten Alkylgruppe, deren Hauptkette 1 bis 4 Kohlenstoffatome aufweist, besteht;
und den Azobenzolen, die durch die folgende chemische Formel (II) dargestellt werden: 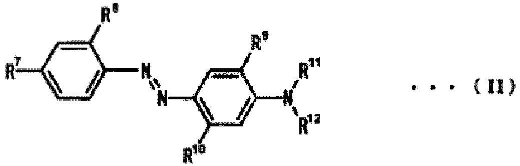 wobei in der chemischen Formel (II) R⁷ und R⁸ gleich oder verschieden sind und aus der Gruppe ausgewählt sind, die aus einem Wasserstoffatom, einer Hydroxygruppe, einer Nitrogruppe, einer Cyanogruppe, einer Halogengruppe und einer Acylaminogruppe besteht; R⁹ und R¹⁰ gleich oder verschieden sind und aus der Gruppe ausgewählt sind, die aus einem Wasserstoffatom, einer Hydroxygruppe und einer Alkylgruppe mit 1 bis 4 Kohlenstoffatomen besteht; R¹¹ und R¹² gleich oder verschieden sind und aus der Gruppe ausgewählt sind, die aus einem Wasserstoffatom, einer Hydroxygruppe, einer Alkylgruppe mit 1 bis 4 Kohlenstoffatomen, einer Phenylgruppe, einer cyanosubstituierten Alkylgruppe, deren Hauptkette 1 bis 4 Kohlenstoffatome aufweist, einer hydroxysubstituierten Alkylgruppe, deren Hauptkette 1 bis 4 Kohlenstoffatome aufweist, und einer acyloxysubstituierten Alkylgruppe, deren Hauptkette 1 bis 4 Kohlenstoffatome aufweist, besteht.

3. Zusammensetzung mit dem Sterilisationsindikator gemäß Anspruch 1, wobei das reduzierende Saccharid aus der Gruppe ausgewählt ist, die aus Stärke, Dextrin, Alginsäure, Carrageen, Gummi arabicum, Agar, Agarose, Galactan, Pektin, Glucose, Galactose, Mannose und Arabinose besteht.

4. Zusammensetzung mit dem Sterilisationsindikator gemäß Anspruch 3, wobei das Derivat ein Salz oder ein Ester ist.

5. Zusammensetzung mit dem Sterilisationsindikator gemäß Anspruch 1, wobei es sich bei dem Polysaccharid um Carrageen, Agar oder Alginsäure, die eine Sulfonsäuregruppe oder eine Carboxygruppe innerhalb ihrer Molekularstruktur aufweist, handelt.

6. Zusammensetzung mit dem Sterilisationsindikator gemäß Anspruch 1, wobei die organische Säure eine aliphatische Carbonsäure oder eine aromatische Carbonsäure ist.

7. Sterilisationsindikator, umfassend eine ihre Farbe verändernde Schicht aus der Zusammensetzung mit dem Sterilisationsindikator gemäß Anspruch 1, die auf ein zu sterilisierendes Substrat oder Produkt aufgetragen ist.

8. Sterilisationsindikator gemäß Anspruch 7, wobei das Substrat aus Papier oder Kunststoff besteht und in Form einer Karte, eines Bandes, einer Folie oder eines Beutels vorliegt.

9. Sterilisationsindikator gemäß Anspruch 7, wobei die ihre Farbe verändernde Schicht mit einer Schutzschicht, die aus einem Kunststofffilm besteht, bedeckt ist.

10. Verwendung der Zusammensetzung mit dem Sterilisationsindikator gemäß Anspruch 1 zum Nachweis der Beendigung der Sterilisation durch den Hochdruckdampf und das Ethylenoxidgas.

## Revendications

1. Composition d'un indicateur de stérilisation à utiliser pour détecter une stérilisation soit par vapeur à haute pression soit par l'oxyde d'éthylène gazeux, comprenant des composés pour changer la couleur en une autre teinte couleur, ladite composition comprenant :
un saccharide réducteur choisi dans le groupe consistant en un monosaccharide réducteur ayant au moins un groupe aldéhyde libre ou un groupe hémiacétal, et un polysaccharide hydrosoluble ayant un monosaccharide réducteur en tant que motif répétitif,
un colorant monoazoïque comprenant au moins un colorant choisi dans le groupe consistant en des benzothiazolylazobenzènes et des azobenzènes,
un acide organique, et
un véhicule d'encre comprenant au moins l'un choisi dans le groupe consistant en éthylcellulose, nitrocellulose, une résine de type butyral, une résine acrylique, une résine polyamide, une résine phénolique et une résine colophane/acide maléique modifiée.

2. Composition de l'indicateur de stérilisation selon la revendication 1, dans laquelle ledit colorant monoazoïque est au moins l'un choisi dans le groupe consistant en les benzothiazolylazobenzènes représentés par la formule chimique (I) suivante : 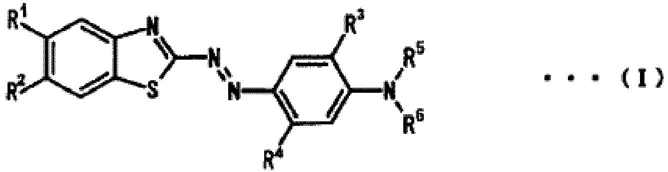 où dans la formule chimique (I), R¹ est choisi dans le groupe consistant en un atome d'hydrogène, un groupe hydroxy, un groupe alkylsulfo avec 1 à 4 atomes de carbone, un groupe alkoxy avec 1 à 4 atomes de carbone, un groupe halogène, un groupe aminoacyle, et un groupe nitro, R² est choisi dans le groupe consistant en un atome d'hydrogène, un groupe hydroxy, un groupe alkylsulfo avec 1 à 4 atomes de carbone, un nitrate, un groupe alkyl avec 1 à 4 atomes de carbone, un groupe alkyl substitué par acyloxy et ayant une chaîne principale avec 1 à 4 atomes de carbone, un groupe alkyl substitué par cyano et ayant une chaîne principale avec 1 à 4 atomes de carbone, un groupe alkyl substitué par alcoxy et ayant une chaîne principale avec 1 à 4 atomes de carbone, et un groupe halogène, R³ et R⁴, identiques ou différents, sont choisis dans le groupe consistant en un atome d'hydrogène, un groupe hydroxy, un groupe alkyl avec 1 à 4 atomes de carbone, un groupe alkoxy avec 1 à 4 atomes de carbone, et un groupe acylamino, R⁵ et R⁶, identiques ou différents, sont choisis dans le groupe consistant en un atome d'hydrogène, un groupe hydroxy, un nitrate, un groupe alkyl avec 1 à 4 atomes de carbone, un groupe alkyl substitué par acyloxy et ayant une chaîne principale avec 1 à 4 atomes de carbone, un groupe alkyl substitué par cyano et ayant une chaîne principale avec 1 à 4 atomes de carbone, un groupe alkyl substitué par alcoxy et ayant une chaîne principale avec 1 à 4 atomes de carbone, et un groupe alkyl substitué par hydroxy et ayant une chaîne principale avec 1 à 4 atomes de carbone,
et les azobenzènes représentés par la formule chimique (II) suivante : 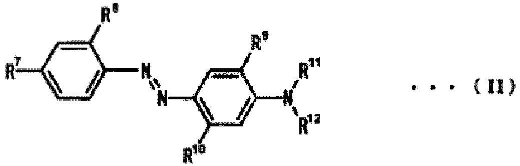 où dans la formule chimique (II), R⁷ et R⁸, identiques ou différents, sont choisis dans le groupe consistant en un atome d'hydrogène, un groupe hydroxy, un groupe nitro, un groupe cyano, un groupe halogène, et un groupe acylamino, R⁹ et R¹⁰, identiques ou différents, sont choisis dans le groupe consistant en un atome d'hydrogène, un groupe hydroxy, et un groupe alkyl avec 1 à 4 atomes de carbone, R¹¹ et R¹², identiques ou différents, sont choisis dans le groupe consistant en un atome d'hydrogène, un groupe hydroxy, un groupe alkyl avec 1 à 4 atomes de carbone, un groupe phényle, un groupe alkyl substitué par cyano et ayant une chaîne principale avec 1 à 4 atomes de carbone, un groupe alkyl substitué par hydroxy et ayant une chaîne principale avec 1 à 4 atomes de carbone, et un groupe alkyl substitué par acyloxy et ayant une chaîne principale avec 1 à 4 atomes de carbone.

3. Composition de l'indicateur de stérilisation selon la revendication 1, dans laquelle ledit saccharide réducteur est choisi dans le groupe consistant en amidon, dextrine, acide alginique, carraghénane, gomme arabique, agar, agarose, galactane, pectine, glucose, galactose, mannose, et arabinose.

4. Composition de l'indicateur de stérilisation selon la revendication 3, dans laquelle ledit dérivé est un sel ou un ester.

5. Composition de l'indicateur de stérilisation selon la revendication 1, dans laquelle ledit polysaccharide est le carraghénane, l'agar ou l'acide alginique ayant un groupe sulfonique ou un groupe carboxylique au sein de sa structure moléculaire.

6. Composition de l'indicateur de stérilisation selon la revendication 1, dans laquelle ledit acide organique est un acide carboxylique aliphatique ou un acide carboxylique aromatique.

7. Indicateur de stérilisation comprenant une couche changeante de couleur de la composition de l'indicateur de stérilisation selon la revendication 1, appliquée sur un substrat ou un produit à stériliser.

8. Indicateur de stérilisation selon la revendication 7, dans lequel ledit substrat est en papier ou en plastique et se présente sous la forme d'une carte, d'un ruban, d'une feuille, ou d'un sac.

9. Indicateur de stérilisation selon la revendication 7, dans lequel ladite couche changeante de couleur est couverte d'une couche protectrice constituée d'un film plastique.

10. Utilisation de la composition de l'indicateur de stérilisation selon la revendication 1 pour détecter l'achèvement de la stérilisation par la vapeur à haute pression et par l'oxyde d'éthylène gazeux.
